(19) **Europäisches Patentamt / European Patent Office / Office européen des brevets**

(11) **EP 2 629 662 B1**

(12) # FASCICULE DE BREVET EUROPEEN

(45) Date de publication et mention de la délivrance du brevet:
**01.06.2022 Bulletin 2022/22**

(21) Numéro de dépôt: **11794532.9**

(22) Date de dépôt: **21.10.2011**

(51) Classification Internationale des Brevets (IPC):
**A61B 5/11** *(2006.01)*     **G01G 19/44** *(2006.01)*

(52) Classification Coopérative des Brevets (CPC):
**A61B 5/4023; A61B 5/1036; A61B 5/11;
G01G 19/44; G01G 23/3742**

(86) Numéro de dépôt international:
**PCT/FR2011/052472**

(87) Numéro de publication internationale:
**WO 2012/052697 (26.04.2012 Gazette 2012/17)**

(54) **PROCEDE, DISPOSITIF ET SYSTEME DE CALCUL DE PARAMETRES L'EQUILIBRE**

VERFAHREN, VORRICHTUNG UND SYSTEM ZUR BERECHNUNG VON GLEICHGEWICHTSPARAMETERN

METHOD, DEVICE, AND SYSTEM FOR CALCULATING PARAMETERS OF BALANCE

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **21.10.2010 FR 1058589**

(43) Date de publication de la demande:
**28.08.2013 Bulletin 2013/35**

(73) Titulaire: **Université de Technologie de Troyes
10010 Troyes Cédex (FR)**

(72) Inventeurs:
- **HEWSON, David James
F-10440 Torvilliers (FR)**
- **DUCHENE, Jacques
F-10440 La Riviere De Corps (FR)**
- **SNOUSSI, Hichem
F-10120 St Andre Les Vergers (FR)**
- **HOGREL, Jean-Yves
F-92120 Montrouge (FR)**
- **LANGERON, Yves
F-10800 Villemereuil (FR)**

(74) Mandataire: **Debay, Damien et al
Debay IP
126 Résidence Elysée 2
18, avenue de la Jonchère
78170 La Celle-Saint-Cloud (FR)**

(56) Documents cités:
**FR-A1- 2 839 148     JP-A- 9 257 556**

- **MICHEL-PELLEGRINO V ET AL: "Effect of aging on the weight transfer phase when stepping-down backwards", GAIT & POSTURE, ELSEVIER, vol. 24, 1 décembre 2006 (2006-12-01), pages S281-S282, XP025013922, ISSN: 0966-6362, DOI: DOI:10.1016/J.GAITPOST.2006.11.192 [extrait le 2006-12-01]**
- **MICHEL-PELLEGRINO V ET AL: "Evaluation of the risk of falling in institution-dwelling elderly: clinical tests versus biomechanical analysis of stepping-up", 2007 ANNUAL INTERNATIONAL CONFERENCE OF THE IEEE ENGINEERING IN MEDICINE AND BIOLOGY SOCIETY : [EMBC '07] ; LYON, FRANCE, 22 - 26 AUGUST 2007 ; [IN CONJUNCTION WITH THE BIENNIAL CONFERENCE OF THE SOCIÉTÉ FRANÇAISE DE GÉNIE BIOLOGIQUE ET MÉDICAL (SFGB, 22 août 2007 (2007-08-22), pages 6121-6124, XP031337625, ISBN: 978-1-4244-0787-3**
- **JONSSON ET AL: "Age-related differences in postural adjustments in connection with different tasks involving weight transfer while standing", GAIT & POSTURE, ELSEVIER, vol. 26, no. 4, 20 septembre 2007 (2007-09-20), pages 508-515, XP022261708, ISSN: 0966-6362, DOI: DOI:10.1016/J.GAITPOST.2006.11.206**

- **SNOUSSI H ET AL: "Reconstructed Phase Spaces of Intrinsic Mode Functions. Application to Postural Stability Analysis", CONFERENCE PROCEEDINGS. ANNUAL INTERNATIONAL CONFERENCE OF THE IEEE ENGINEERING IN MEDICINE AND BIOLOGY SOCIETY (IEEE CAT. NO. 06CH37748); 30 AUG.-3 SEPT. 2006; NEW YORK, NY, USA, IEEE, PISCATAWAY, NJ, USA, 30 août 2006 (2006-08-30), pages 4584-4589, XP031390537, ISBN: 978-1-4244-0032-4**
- **MICHEL ET AL: "2.19 The effect of age on dynamic postural equilibrium when stepping up", GAIT & POSTURE, ELSEVIER, AMSTERDAM, NL, vol. 21, 1 juin 2005 (2005-06-01), pages S9-S10, XP005032650, ISSN: 0966-6362**
- **MICHEL ET AL: "2.19 The effect of age on dynamic postural equilibrium when stepping up", GAIT & POSTURE, ELSEVIER, AMSTERDAM, NL, vol. 21, 1 June 2005 (2005-06-01), pages S9-S10, XP005032650, ISSN: 0966-6362**

**Description**

La présente invention concerne le domaine de l'évaluation de la qualité de l'équilibre. La présente invention concerne plus particulièrement un dispositif, un système et un procédé pour le calcul de paramètres d'évaluation de la qualité de l'équilibre. L'invention permet en particulier un suivi de l'évolution de cette qualité de l'équilibre, notamment chez le sujet âgé, notamment de façon à permettre de prévenir les risques de chute en observant cette évolution.

La chute des personnes dans leur environnement quotidien est un problème majeur de santé publique en raison de leur fréquence et de leurs conséquences médicales et sociales, notamment et surtout chez le sujet âgé et chez l'adulte handicapé. Intuitivement, la notion d'équilibre semble naturellement liée au risque de chute. La pertinence de cette hypothèse a déjà été vérifiée dans des études antérieures. La pertinence de l'invention concerne également la faisabilité technologique et surtout l'acceptabilité socio-économique. Actuellement, la détection des personnes à risque de chute est peu appliquée et peu opérante, souvent éloignée de la vie courante ou inacceptable humainement.

Il est connu dans l'art antérieur des systèmes de laboratoire tels que des plateformes de forces pour l'étude de stabilogrammes, c'est-à-dire l'étude de la position du centre de pression (correspondant à la projection verticale du centre de gravité). Cependant, ces systèmes sont sophistiqués, encombrants et coûteux. Il n'est pas possible d'envisager leur utilisation chez le patient. Il n'existe en fait pour l'instant aucun système simple qui puisse être utilisé à domicile. Il existe donc un besoin pour un dispositif d'évaluation de l'équilibre qui puisse être utilisé en routine chez les patients, de faible coût, pour extraire les informations nécessaires en évitant de perturber l'environnement habituel de vie.

Un autre problème dans le domaine concerne les méthodes d'analyse pour l'évaluation de l'équilibre. En effet, il est connu diverses méthodes d'analyse de l'équilibre mais aucune ne fournit réellement de paramètres suffisamment pertinents et mesurables facilement à l'aide de dispositifs simples et peu coûteux. De plus, les méthodes connues reposent souvent sur des protocoles de mesure relativement complexes qui nécessitent généralement l'assistance d'un opérateur qualifié pour guider le sujet au cours du protocole.

Dans ce contexte, il est intéressant de proposer une solution pour l'évaluation de la qualité de l'équilibre qui repose sur des dispositifs simples et peu coûteux, utilisable facilement à domicile et ne requérant pas de protocole complexe.

La présente invention a pour but de pallier au moins certains inconvénients de l'art antérieur en proposant un procédé d'évaluation de l'équilibre efficace et facile à mettre en oeuvre. L'art antérieur pertinent inclut: JP H-9257556; "Effect of aging on the weight transfer phase when stepping-down backwards" de Michel-Pellegrino et al., Gait & Posture 24S (2006) pp. S281-S282; "Evaluation of the risk of falling in institution-dwelling elderly: clinical tests versus biomechanical analysis of stepping-up" de Michel-Pellegrino et al., Proc. 2007 annual international conférence of the IEEE Engineering in Medicine and Biology Society, pp. 6121-6124; "Age-related différences in postural adjustments in connection with différent tasks involving weight transfer while standing" de Jonsson et al., Gait & Posture 26 (2007) pp. 508-515; "Reconstructed Phase Spaces of Intrinsic Mode Functions. Application to Postural Stability Analysis" de Snoussi et al., Proc. 28th Annual International Conference of the IEEE Engineering in Medicine and Biology Society 2006, pp. 4584-4589; FR 0205382; et en particulier "The effect of age on dynamic postural equilibrium when stepping up" de Michel et al., Gait & Posture, 21 (2005) pp. S9-S10.

Ce but est atteint par un procédé de calcul de paramètres d'évaluation de l'équilibre d'un sujet selon la revendication indépendante 1.

Selon l'invention, l'étape de calcul des paramètres concerne au moins une sigmoïde et/ou au moins un pic dans la montée des valeurs mesurées par les capteurs de pression, lors de la montée du sujet sur le dispositif.

Selon une particularité, l'étape de calcul des paramètres pertinents dans l'évaluation comporte une étape de calcul de la somme des valeurs mesurées par chacun des capteurs de pression.

Selon une autre particularité, le procédé comporte une étape de diffusion, par des moyens de diffusion, d'informations relatives au moins à une invitation du sujet à monter sur le dispositif, l'étape de calcul des paramètres pertinents dans l'évaluation comportant une étape de calcul d'au moins un paramètre relatif au délai écoulé entre l'invitation à monter et la montée du sujet sur le dispositif.

Selon une autre particularité, l'étape de calcul des paramètres pertinents dans l'évaluation comporte une étape de calcul d'au moins un paramètre relatif à la vitesse de montée des valeurs mesurées par les capteurs de pression lors de la montée du sujet sur le dispositif.

Selon une autre particularité, l'étape de calcul des paramètres pertinents dans l'évaluation est précédée d'une étape de calcul d'une phase de montée des valeurs mesurées par les capteurs de pression, lors de la montée du sujet sur le dispositif, cette phase de montée étant déterminée comme la période pendant laquelle lesdites valeurs se situent entre 10% et 90% d'une valeur de référence atteinte une fois le sujet monté sur le dispositif, au moins une partie desdits paramètres calculés correspondant à des valeurs calculées à partir de cette phase de montée .

Selon une autre particularité, l'étape de calcul des paramètres pertinents dans l'évaluation est précédée d'une étape de calcul d'une phase, dite de stabilité , des valeurs mesurées par les capteurs de pression, lorsque le sujet est monté sur le dispositif, cette phase de stabilité étant déterminée comme la période démarrant à un délai déterminé , dit d'attente, après que lesdites valeurs mesurées aient atteint 90% de la valeur de référence et se terminant à un délai déterminé, dit de stabilité, après ledit délai d'attente , l'étape de calcul des paramètres pertinents dans l'évaluation comprenant au moins une étape de calcul de paramètres concernant cette phase de stabilité

Selon une autre particularité, l'étape de calcul des paramètres concernant la phase de stabilité comporte une étape de calcul du barycentre des forces verticales mesurées par chacun des capteurs de pression pour déterminer la position du centre de pression correspondant à la projection du centre de gravité du sujet sur le plateau .

Selon une autre particularité, l'étape de calcul de la position du centre de pression au cours du temps, pendant la phase de stabilité , fournit un stabilogramme permettant la mise en œuvre d'une étape de décomposition modale pour en extraire des fonctions modales intrinsèques, les paramètres concernant cette phase de stabilité comprenant au moins un paramètre déterminé sur ces fonctions modales intrinsèques.

Selon une autre particularité, le procédé comporte au moins une étape de transmission des valeurs mesurées et/ou des paramètres calculés, par des moyens de communication du dispositif , vers au moins un terminal communicant et/ou au moins un serveur de centralisation, via au moins un réseau de communication .

Selon une autre particularité, le procédé comporte au moins une étape de stockage des valeurs mesurées et/ou des paramètres calculés dans des moyens de mémorisation.

Selon une autre particularité, le procédé comporte au moins une étape de calcul de comparaison des valeurs mesurées et/ou des paramètres calculés, stockés dans les moyens de mémorisation, pour au moins deux évaluations déterminées d'un même sujet, afin de permettre un suivi de l'évolution de la qualité de l'équilibre.

Un autre but de la présente invention est de pallier au moins certains inconvénients de l'art antérieur en proposant un dispositif d'évaluation de l'équilibre qui soit peu coûteux et qui puisse être installé facilement à domicile.

Ce but est atteint par un dispositif d'évaluation de l'équilibre d'un sujet selon la revendication indépendante 9. Il comporte un plateau destiné à accueillir les pieds d'un sujet et monté sur une pluralité de capteurs de pression mesurant des forces verticales appliquées sur le plateau , et des moyens de traitement de données agencés pour la mise en œuvre du procédé selon l'invention,

Selon une autre particularité, le plateau possède des dimensions adaptées à la taille moyenne des pieds des sujets, de façon à s'affranchir de problèmes liés à la position des pieds des sujets sur le plateau lors des mesures.

Selon une autre particularité, le dispositif comporte des moyens d'affichage positionnés sur le dispositif de sorte qu'un sujet debout sur le plateau puisse voir l'affichage, de façon à ce que la posture des sujets utilisant le dispositif soit standardisée.

Selon une autre particularité, les moyens de traitement de données comprennent des moyens de mémorisation pour la mise en œuvre de l'étape de stockage des valeurs mesurées et/ou des paramètres calculés.

Un autre but de la présente invention est de pallier au moins certains inconvénients de l'art antérieur en proposant un système d'évaluation de l'équilibre qui soit peu coûteux et qui permette une centralisation des données recueillies à domicile.

Ce but est atteint par un système d'évaluation de l'équilibre d'un sujet selon la revendication indépendante 12. Il comporte un dispositif comprenant un plateau destiné à accueillir les pieds d'un sujet et monté sur une pluralité de capteurs de pression mesurant des forces verticales appliquées sur le plateau , et des moyens de traitement de données recueillant dynamiquement les valeurs mesurées par les capteurs de pression au moins avant et pendant la montée du sujet sur le dispositif et comprenant des moyens de communication de données agencés pour transmettre les valeurs mesurées vers au moins un terminal communicant comprenant des moyens de communication agencés pour recevoir les valeurs mesurées par le dispositif et les transmettre à au moins un serveur de centralisation dont des moyens de traitement de données sont agencés pour pour la mise en œuvre du procédé selon l'invention.

Ce but est également atteint par un système d'évaluation de l'équilibre d'un sujet selon la revendication indépendante 13. Il comprend un dispositif d'évaluation de l'équilibre d'un sujet, comportant un plateau destiné à accueillir les pieds d'un sujet et monté sur une pluralité de capteurs de pression mesurant des forces verticales appliquées sur le plateau , et des moyens de traitement de données recueillant dynamiquement les valeurs mesurées par les capteurs de pression au moins avant et pendant la montée du sujet sur le dispositif et comprenant des moyens de communication de données agencés pour transmettre les valeurs mesurées vers au moins un terminai communicant comprenant des moyens de communication agencés pour recevoir les valeurs mesurées par le dispositif et des moyens de traitement de données agencés pour la mise en oeuvre du procédé selon l'invention, les moyens de communication de ce terminal communicant étant également agencés pour transmettre les valeurs mesurées et/ou les paramètres calculés vers au moins un serveur de centralisation.

D'autres particularités et avantages de la présente invention apparaîtront plus clairement à la lecture de la description ci-après, faite en référence aux dessins annexés, dans lesquels :

- la figure 1 représente un système d'évaluation de l'équilibre selon certains modes de réalisation de l'invention,
- la figure 2 représente un dispositif d'évaluation de l'équilibre selon certains modes de réalisation de l'invention, avec un exemple d'implémentation des moyens de traitement de données,

- les figures 3A et 3B montrent des courbes de valeurs recueillies lors d'une évaluation selon certains modes de réalisation de l'invention, avec diverses phases identifiées et divers paramètres à calculer,
- la figure 4 représente un procédé d'évaluation de l'équilibre selon certains modes de réalisation de l'invention,
- la figure 5 montre un stabilogramme obtenu par des calculs selon certains modes de réalisation de l'invention.

La présente invention concerne un procédé, un dispositif et au moins un système d'évaluation de la qualité de l'équilibre. On parlera plus simplement ici d'évaluation de l'équilibre.

La présente invention propose une approche originale du monitorage non invasif du comportement lié au risque de chute. Intuitivement la notion d'équilibre semble naturellement liée au risque de chute. La pertinence de cette hypothèse a déjà été vérifiée dans des études antérieures.

La plupart des études prospectives sont orientées vers l'identification des facteurs prédictifs majeurs dans les groupes à haut risque de chute. Les facteurs qui reviennent le plus souvent incluent une faiblesse musculaire, une chute précédente, ou des problèmes d'équilibre et de marche. De plus, d'autres facteurs cités comme aggravants en termes de risque de chute, tels que des problèmes visuels, vestibulaires, ou proprioceptifs, peuvent se manifester par un problème d'équilibre. Dans la plupart de ces études, l'équilibre est évalué en utilisant des tests cliniques et biomécaniques. Bien que ces tests aient montré leur capacité à identifier des risques de chute dans l'année, ils ne peuvent pas identifier les changements progressifs. De ce fait, ils sont inadaptés à un test quotidien. De même, une simple mesure biomécanique de balancement et quelques mesures dérivées comme la zone et le type de déplacement du centre de pression ont su prévoir des chutes. Par contre, ces mesures n'ont jamais été intégrées au domicile.

De plus, il existe des systèmes de laboratoire tels que des plates-formes de forces pour l'étude des stabilogrammes, mais il n'existe aucun système simple au domicile. Il y avait donc nécessité de développer des outils de faible coût pour extraire les informations nécessaires, de préférence en perturbant au minimum l'environnement habituel de vie.

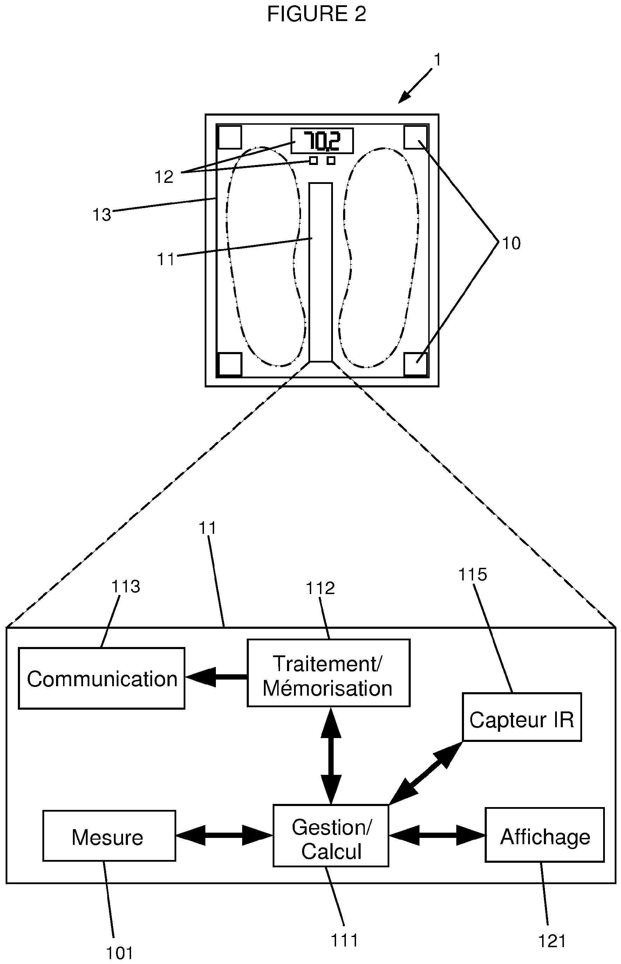

Une originalité de certains modes de réalisation de la présente invention est qu'ils s'appuient sur un objet usuel : le pèse-personne. En effet, certains modes de réalisation de l'invention sont basés sur l'intégration dans un pèse-personne de moyens permettant d'évaluer la qualité de l'équilibre. Le dispositif (1) d'évaluation de l'équilibre est alors un pèse-personne qui comporte un plateau (13) destiné à accueillir les pieds d'un sujet et monté sur une pluralité de capteurs (10) de pression mesurant des forces verticales appliquées sur le plateau (13), généralement avec des moyens d'affichage (12), et modifié pour comporter des moyens de traitement de données (11) agencés pour la mise en œuvre du procédé selon divers modes de réalisation de l'invention. Les capteurs (10) seront de préférence au nombre de quatre, de préférence répartis à proximité des coins du plateau (13), comme représenté sur la figure 2, pour une bonne mesure des forces verticales (le poids exercé par le sujet se tenant debout) et une bonne stabilité du plateau du dispositif. Néanmoins, on peut augmenter ou réduire le nombre de capteurs (10) tout en préservant une mesure fiable (des plateaux à seulement 3 capteurs ont déjà été utilisés avec succès). Un tel dispositif simple permet la mise en œuvre d'un protocole simple et peu risqué pour le sujet, que les inventeurs ont découvert qu'il pouvait être suffisamment pertinent pour l'évaluation de l'équilibre s'il s'accompagnait de la détection d'au moins un événement et de la mesure d'au moins un paramètre identifié par les inventeurs, comme détaillé ci-après. Ce protocole requiert simplement que le sujet monte sur le plateau (13) de mesure du dispositif (1), de la même manière qu'il le fait pour se peser en général.

Le plateau (13) possède de préférence des dimensions adaptées à la taille moyenne des pieds des sujets, de façon à s'affranchir de problèmes liés à la position des pieds des sujets sur le plateau lors des mesures. Par exemple, un plateau sensiblement carré d'environ 30 cm de côté ou rectangulaire permet que les pieds des sujets soient toujours correctement placés par rapport aux capteurs (10). D'une manière générale, les dimensions d'un côté du plateau (13) pourront être compris entre 15 et 70 cm, de préférence de l'ordre de 25 à 40 cm.

De plus, les moyens d'affichage (12) d'un pèse personne sont généralement positionnés sur le dispositif (1) de sorte qu'un sujet debout sur le plateau (13) puisse voir l'affichage. Le dispositif (1) reprenant cette disposition dans certains modes de réalisation permet que la posture des sujets utilisant le dispositif (1) soit standardisée. Tous les sujets se tiennent debout, généralement les bras le long du corps, en regardant l'affichage qui se situe à peu près au niveau de leurs pointes de pieds.

Enfin, divers modes de réalisation de l'invention sont mis en œuvre grâce à une invitation de la personne à monter sur le dispositif. Le dispositif (généralement le pèse-personne) comporte des moyens de diffusion pour fournir au sujet des informations relatives à une invitation à monter dessus. Le pèse-personne affiche par exemple une telle invitation (par exemple une LED verte ou sur l'écran d'affichage), le sujet monte sur le plateau, attend l'affichage de son poids, puis redescend (par exemple suite à l'affichage d'une invitation à descendre, par exemple une LED rouge ou une invitation sur l'écran d'affichage, voire simplement l'affichage du poids du sujet qui permet à ce dernier de savoir qu'il peut descendre). On obtient ainsi un protocole simple qui ne modifie pas les habitudes du sujet, ce qui peut être particulièrement avantageux pour certains sujets particuliers.

Ainsi, divers modes de réalisation de l'invention sont particulièrement adaptés à leur utilisation à domicile car les sujets disposent en apparence simplement d'un pèse-personne sur lequel ils montent, par exemple quotidiennement, pour mesurer leur poids, mais qui permet en même temps de suivre l'évolution de la qualité de leur équilibre. L'invention permet bien entendu qu'un seul dispositif suive plusieurs personnes, grâce à la gestion de plusieurs profils à l'aide des moyens (11) de traitement de données et des moyens d'affichage (12).

Dans certains modes de réalisation, ce dispositif (1) placé au domicile des sujets permet un suivi "en continu", d'abord grâce à un apprentissage, puis grâce à une évaluation des déviations par rapport à la situation de référence apprise.

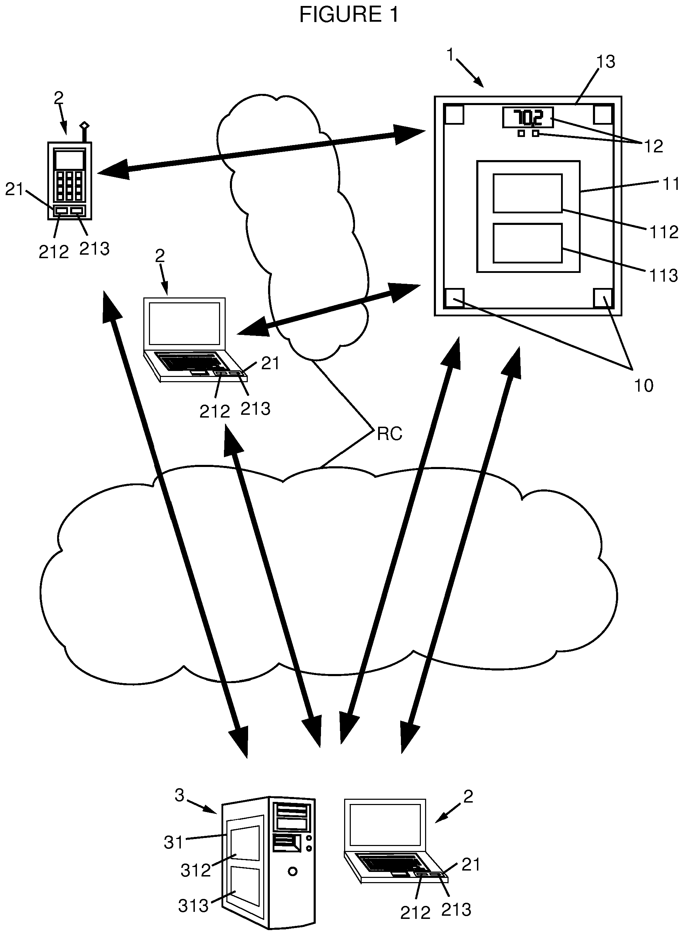

La figure 1 représente un système d'évaluation de l'équilibre selon divers modes de réalisation. La présente invention peut en effet être mise en œuvre sur un seul dispositif d'évaluation (1) autonome ou, de préférence, être mise en œuvre dans un système comprenant le dispositif d'évaluation (1) et au moins un terminal communicant (2) et/ou au moins un serveur de centralisation (3). Selon divers modes de réalisation, les moyens de traitement de données (11) de ce dispositif (1) seront donc agencés pour réaliser tout ou partie des étapes du procédé selon l'invention. Lorsque le dispositif n'effectue pas toutes les étapes de façon autonome, il sera alors seulement équipé des moyens d'affichage (12) éventuels, du plateau (13) et des capteurs (10) et de moyens de traitement (11) agencés uniquement pour recueillir les données des capteurs, contrôler les moyens d'affichage (12) éventuels, recueillir les valeurs mesurées par les capteurs (13), et pour contrôler les moyens de communication (113) permettant la transmission des données acquises. Dans ce cas, le dispositif (1) sera équipé de moyens de communication (113) sur au moins un réseau de communication (RC) pour transmettre les données (valeurs mesurées et/ou paramètres calculés) vers au moins un terminal communicant (2) et/ou au moins un serveur de centralisation (3). Le dispositif (1), selon diverses variantes, pourra alors éventuellement effectuer ou non au moins une partie des calculs pour la mise en œuvre du procédé. Ce terminal (2) et/ou ce serveur (3) pourra (ou pourront) réaliser les étapes du procédé (notamment les calculs) qui n'auront pas été réalisées par le dispositif (1). En variante, les moyens de transmission (113) de données du dispositif (1) peuvent être remplacés par des moyens de stockage amovible (comme par exemple un lecteur de carte mémoire pour le stockage des données). Les données ainsi stockées pourront être alors transmises si nécessaire à un terminal (2) équipé d'un lecteur pour leur traitement. Néanmoins, les modes de réalisation où le dispositif (1) possède des moyens de communication (113) pour transmettre ces données via un réseau de communication (RC) sont préférés pour des raisons pratiques.

Ainsi, dans la figure 1 apparaissent en fait plusieurs modes de réalisation possibles :
Soit le dispositif (1) est agencé pour transmettre directement ses données vers au moins un terminal communicant (2) et/ou au moins un serveur de centralisation (3), grâce par exemple à des moyens de communication à longue distance (par exemple une connexion internet, via un réseau local ou directement, ou encore par un réseau téléphonique, notamment de dernière génération), filaire ou non, comme représenté par la flèche la plus à droite sur la figure 1 qui va directement du dispositif vers le terminal (2) et/ou le serveur (3). Le terminal communicant (2) pourra par exemple être l'ordinateur d'un médecin qui suit l'évolution de l'équilibre du sujet (ou de plusieurs sujets) et qui reçoit ainsi régulièrement les données acquises par le(s) dispositifs) (1) d'évaluation, Le serveur (3) de centralisation pourra être un serveur d'un fournisseur de service qui centralise les données et leur traitement pour une pluralité de sujets et/ou pour une pluralité de médecins suivant des sujets par exemple.

Soit le dispositif (1) est agencé pour transmettre ses données vers au moins un terminal communicant (2), comme par exemple un téléphone mobile ou un ordinateur, grâce par exemple à des moyens de communication à courte distance (comme par exemple une connexion Bluetooth, qui est préférée ici pour sa simplicité et sa relative universalité, ou une connexion filaire) ou même à longue distance (comme par exemple une connexion filaire ou wifi), comme représenté par la flèche en haut à gauche sur la figure 1. Le terminal communicant (2) sera dans ce cas celui d'un sujet qui a accès au dispositif (1) pour le suivi de son équilibre. Ce terminal (2) du sujet est alors agencé, soit pour transmettre directement les données (avec, en variante ici également, la possibilité d'un stockage sur un support amovible) transmettre les données vers au moins un terminal communicant (2) et/ou au moins un serveur de centralisation (3), soit pour d'abord traiter les données en mettant en œuvre au moins une partie des étapes du procédé (notamment les calculs détaillés ici) avant de les transmettre (ou les stocker dans un support amovible). Cette seconde transmission du terminal (2) du sujet vers un autre terminal (2) ou un serveur (3) pourra alors être faite par exemple grâce à des moyens de communication à longue distance (par exemple une connexion internet, via un réseau local ou directement, ou encore par un réseau téléphonique, notamment de dernière génération), filaire ou non, comme représenté par la flèche en bas à gauche sur la figure 1. Ce deuxième terminal (2) communicant qui reçoit les données du terminal (2) communicant du sujet pourra par exemple être l'ordinateur d'un médecin qui suit l'évolution de l'équilibre du sujet (ou de plusieurs sujets) et qui reçoit ainsi régulièrement les données acquises par le(s) dispositif(s) (1) d'évaluation. Le serveur (3) de centralisation pourra être un serveur d'un fournisseur de service qui centralise les données et leur traitement pour une pluralité de sujets et/ou pour une pluralité de médecins suivant des sujets par exemple.

On comprend donc que la présente invention repose sur au moins un dispositif (1) d'évaluation de l'équilibre qui permet au moins des mesures grâce à un protocole simple et que les calculs qui sont ensuite mis en œuvre sur les données acquises peuvent être réalisés en local sur le dispositif ou à distance sur divers types de terminal ou serveur. Les exemples illustratifs fournis ici ne sont bien entendu pas limitatifs et il apparaitra à la lecture de la présente demande que l'invention peut être mise en oeuvre à l'aide d'autres types de terminaux que ceux donnés ici à titre d'exemple, à l'exception du dispositif (1) d'évaluation qui devra au moins comporter les moyens décrits ici (les moyens de traitement intégrant tout ou partie des fonctions décrites ici). De même, on parle ici de moyens de diffusion (12) qui sont de préférence des moyens d'affichage (12) pour guider l'utilisateur mais il apparaitra à la lecture de la présente demande que de tels moyens peuvent prendre diverses formes ou être remplacés par des moyens audio ou tout autre équivalent permettant de délivrer des informations au sujet qui utilise le dispositif (1), car il s'agit uniquement de le guider dans le protocole de mesure qui est très simple. Par exemple les figures 1 et 2 représentent les moyens d'affichage (12) comme un écran affichant des informations (le poids en kilogrammes par exemple) et des LED (diodes électroluminescentes) indiquant par exemple que l'appareil est en veille ou en marche ou en attente de la montée ou de la descente du sujet (selon divers codes possibles), mais on comprendra que ce ne sont que des exemples illustratifs et que l'un ou l'autre de ces moyens peut être omis ou remplacé par d'autres moyens. De plus, les figures 1 et 2 représente les moyens de traitement (11) comme intégrant des moyens (112) de traitement et de mémorisation de données. On comprendra qu'il peut s'agir en fait d'une mémoire tampon, par exemple de type volatile servant uniquement à un stockage provisoire pour la transmission de données (ou, dans les variantes mentionnées précédemment, l'enregistrement dans un support amovible). Les moyens de traitement de données (11) sont donc agencés au moins pour la mise en œuvre du protocole de mesure, avec une invitation à monter, une éventuelle invitation à attendre, un éventuel affichage du poids et une éventuelle invitation à descendre (chaque éventualité pouvant par exemple être remplacée par l'absence d'affichage ou un affichage neutre). Cette mise en oeuvre du protocole nécessite le contrôle de la mesure par les capteurs (10), le contrôle de l'affichage, et le contrôle de la mémorisation et/ou la transmission. La figure 2 représente par exemple un module (111) de gestion/calcul qui contrôlera ces moyens du dispositif (1), par exemple grâce à un module spécifique (101, 121, 112, 113) pour chaque moyen, comme indiqué sur la figure 2. D'autre part, le dispositif (1) peut être équipé d'un capteur infra-rouge détectant la présence du sujet et démarrant ainsi le protocole. Un module spécifique (115) est représenté pour le contrôle d'un tel capteur, mais ce module et ce capteur peuvent être omis ou remplacé par d'autres moyens de détection, Il est possible par exemple qu'il faille appuyer sur un bouton ou le plateau pour démarrer le protocole. Comme mentionné précédemment, les moyens de traitement (11), notamment le module de gestion/calcul dans les modes de réalisation où il est présent, est agencé pour effectuer au moins une partie des étapes du procédé selon l'invention. Les étapes nécessaires au protocole seront forcément gérées par les moyens de traitement, mais les calculs pourront être déportés en tout ou partie sur au moins un terminal (2) communicant et/ou au moins un serveur (3) de centralisation.

Le dispositif (1) pourra, dans certains modes de réalisation être agencé comme représenté sur l'exemple de la figure 2. Dans cet exemple, les moyens de traitement de données (11) du dispositif (1) sont représentés selon un agencement en différents modules (111, 101, 121, 112, 113, 115) chargés des différentes opérations à réaliser. On comprendra néanmoins que l'invention peut prévoir divers types d'agencement, comme par exemple un seul module opérationnel cumulant l'ensemble des fonctions décrites ici. Ces moyens de traitement (11) sont des ressources électroniques de préférence intégrées directement dans le pèse-personne, comme par exemple des cartes électroniques, mais peuvent en fait lui être associé, au moins en partie (par exemple en étant déportés dans un autre dispositif communicant avec le pèse-personne grâce aux moyens de communication). Selon divers modes de réalisation, on pourra prévoir plusieurs cartes différentes ou un seul circuit intégrant toutes les fonctionnalités. Par exemple, on pourra avoir une première carte qui assure le déroulement du protocole et fournit les données capteurs, une deuxième carte qui permet la gestion des données envoyées par la première carte, leur stockage temporaire, puis leur envoi au travers d'une interface sans fil vers un système de réception, que ce soit un PC ou un téléphone portable.

Comme mentionné précédemment, l'invention concerne, entre autres, un procédé de calcul de paramètres évaluation de l'équilibre d'un sujet qui est mis en œuvre à l'aide d'au moins un dispositif (1) selon l'invention. Ce procédé comporte entre autres les étapes suivantes :

Mesure (51), par les capteurs (10) de pression, des forces verticales appliquées sur le plateau (13), au moins pendant la montée du sujet sur le dispositif (1),
- Calcul (53), à partir de ces valeurs mesurées, de paramètres pertinents dans l'évaluation.

L'étape de calcul (53) peut permettre de calculer au moins un paramètre relatif à au moins une inflexion dans la montée des valeurs mesurées par les capteurs (10) de pression lors de la montée du sujet sur le dispositif (1). Dans certains modes de réalisation le procédé comporte au moins une étape de diffusion, par exemple une étape d'affichage (50), sur des moyens de diffusion, par exemple les moyens d'affichage (12), d'informations relatives au moins à une invitation du sujet à monter sur le dispositif (1).

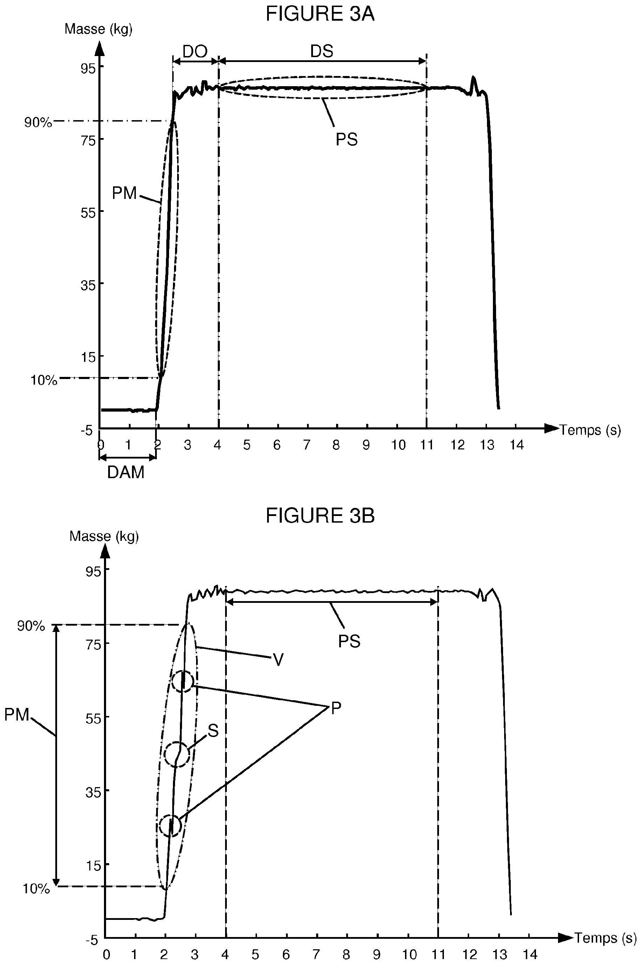

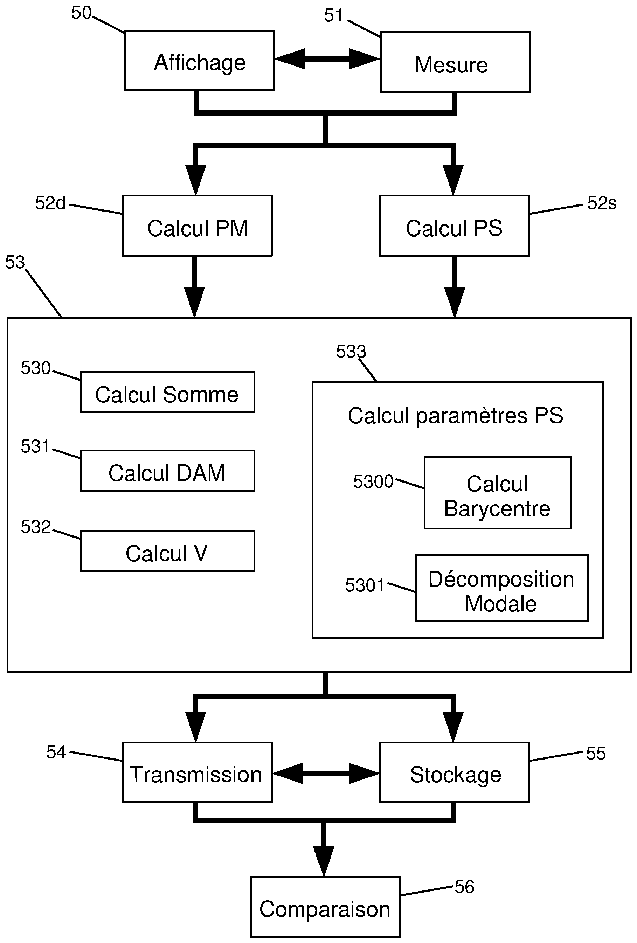

Au cours du protocole qui, pour rappel, ne nécessite que la montée du sujet, de préférence suite à invitation par le dispositif (1), puis de préférence un maintien de la posture debout quelques secondes, le dispositif (1) recueillie les valeurs mesurées par les capteurs (10), c'est-à-dire les forces verticales appliquées sur chacun des capteurs (10) sous le plateau (13). Les figures 3A et 3B montrent un exemple de la somme des mesures de quatre capteurs au cours d'un protocole où le sujet monte, attend, puis redescend du dispositif (1). L'étape de calcul (53) des paramètres pertinents dans l'évaluation comporte, pour la plupart des modes de réalisation, une étape (530) de calcul de la somme des valeurs mesurées par chacun des capteurs (10) de pression. La figure 3A ne montre pas d'inflexion dans la montée, et la figure 3B montre plusieurs types d'inflexions possibles. En effet, les courbes mesurées pour certains sujets ne présentent pas d'inflexion. En revanche, certains sujets, lorsqu'ils montent sur un pèse personne, donnent des à-coups assez importants du fait d'un mauvais équilibre. Cela se traduit dans les mesures par un pic dans la montée, correspondant à une inflexion brutale. D'autres sujets ont des déséquilibres moins importants qui se traduisent par une légère inflexion sous la forme d'une sigmoïde en général. D'autres sujets présentent les deux types d'inflexions (légère et brutale). Ainsi, selon l'invention, le calcul (53) des paramètres relatifs à au moins une inflexion concerne au moins une sigmoïde (S) et/ou au moins un pic (P) dans la montée des valeurs mesurées par les capteurs (10) de pression, lors de la montée du sujet sur le dispositif (1).On parle ici d'inflexion pour regrouper les deux notions de pics et de sigmoïdes, pour simplifier le propos. De même, le terme sigmoïde n'est pas limitatif et on devra le comprendre comme désignant une inflexion plus lente et de plus faible amplitude que celle désignée sous le terme de pic. Les paramètres relatifs à ces inflexions pourront concerner la présence et/ou les caractéristiques spécifiques des inflexions (pics et/ou sigmoïdes). Comme caractéristiques spécifiques, on peut par exemple calculer l'amplitude de l'inflexion, sa durée, etc. En effet, les inventeurs de la présente invention ont découvert que, même lors d'un protocole aussi simple qu'une montée du sujet sur un plateau de mesure, tel que celui d'un pèse-personne par exemple, la présence d'une inflexion dans la montée des valeurs mesurées était un paramètre particulièrement pertinent pour l'évaluation de l'équilibre du sujet. Ainsi, certains modes de réalisation de la présente invention sont des applications techniques de cette découverte. Le procédé, le dispositif ou le système l'invention utilise alors au moins un algorithme de détection d'une inflexion dans la montée des valeurs mesurées par les capteurs. De plus, cet algorithme peut être associé ou comporter au moins un algorithme de calcul d'au moins un paramètre relatif à cette inflexion, comme par exemple ceux mentionnés ci-dessus. Ainsi, il est fait de préférence appel à au moins un calcul d'au moins un paramètre relatif à au moins une inflexion dans la montée des valeurs mesurées par les capteurs (10) de pression lors de la montée du sujet sur le dispositif (1), ce paramètre étant représentatif de la présence et/ou de caractéristiques spécifiques de cette inflexion, comme détaillé ci-dessus.

D'une manière générale, on comprend de la présente demande que des algorithmes implémentés dans les moyens de traitement (dans le dispositif de mesure ou dans un terminal qui lui est associé) pourront être prévus pour réaliser les détections d'événements et de paramètres relatifs à ces événements décrits dans la présente demande (phase de montée, délais avant montée, inflexion, vitesse de montée, phase de stabilité, etc.)

Dans certains modes de réalisation du procédé, l'étape de calcul (53) des paramètres pertinents dans l'évaluation est précédée d'une étape de calcul (52d) d'une phase de montée (PM) des valeurs mesurées par les capteurs (10) de pression, lors de la montée du sujet sur le dispositif (1). La figure 3A montre cette phase de montée (PM), par une ellipse en pointillé sensiblement verticale (à gauche, sur la partie montante de la courbe) et montre une phase dite de stabilité (PS) par une ellipse en pointillé sensiblement horizontale (à droite, sur la partie en plateau de la courbe).

La phase de montée (PM) est, selon l'invention, déterminée comme la période pendant laquelle les valeurs mesurées par les capteurs se situent entre 10% et 90% d'une valeur de référence atteinte une fois le sujet monté sur le dispositif (1). Cette valeur de référence pourra correspondre au poids calculé du sujet, prise sur le plateau (valeur maximale) des valeurs mesurées par les capteurs. Lors du calcul de la valeur de référence, certains modes de réalisations prévoient de préférence un filtrage des signaux rapides tels que des oscillations courtes en fin de montée, pour s'affranchir des erreurs dues à des maxima locaux. Par exemple, selon diverses variantes, cette valeur maximum sera calculée sur une courbe fortement lissée (par exemple à l'aide d'une moyenne calculée sur une fenêtre glissante) ou sera calculée par une moyenne sur plusieurs secondes, à un délai suffisamment long après le début de la montée. D'autres méthodes pour obtenir une valeur de référence fiable sont possibles et à la portée de l'homme de métier. Au moins une partie desdits paramètres calculés correspond à des valeurs calculées au cours de cette phase de montée (PM). Dans certains modes de réalisation du procédé, l'étape de calcul (53) des paramètres pertinents dans l'évaluation est précédée d'une étape de calcul (52s) d'une phase, dite de stabilité (PS), des valeurs mesurées par les capteurs (10) de pression, lorsque le sujet est monté sur le dispositif (1). On parle de phase de stabilité car les valeurs de pression mesurée par les capteurs augmentent au cours de la montée du sujet sur le dispositif et se stabilisent à une valeur plateau. Cette stabilité est relative car, les valeurs continuent à osciller en fonction de la qualité de l'équilibre du patient, ce qui permet d'ailleurs de calculer un stabilogramme et divers paramètres pertinents comme détaillé dans la présente demande. Dans certains modes de réalisation, cette phase de stabilité (PS) est déterminée comme la période démarrant à un délai déterminé (DO), dit d'attente, après que lesdites valeurs mesurées aient atteint 90% de la valeur de référence et se terminant à un délai (DS) déterminé, dit de stabilité, après ledit délai d'attente (DO). Dans certains modes de réalisation, l'étape de calcul (53) des paramètres pertinents dans l'évaluation pourra comporter au moins une étape de calcul (533) de paramètres concernant cette phase de stabilité (PS). Le délai d'attente (DO) est de l'ordre d'une seconde (compris entre 0,5 et 2 secondes, par exemple 1,5 seconde) et le délai de stabilité (DS) est de l'ordre de 5 à 10 secondes (compris entre 3 et 20 secondes). La figure 3B montre à nouveau ces 2 phases (PS, PM) en indiquant par une flèche verticale l'amplitude (10 à 90%) prise pour la phase de montée (PM) et l'intervalle de temps pris (4 à 11 s) pour la phase de stabilité (PS). On comprend donc que les calculs font intervenir des valeurs paramétrées comme les délais d'attente (DO) et de stabilité (DS) et des valeurs mesurées (comme la valeur de référence, par exemple le maximum au plateau de la courbe, pour en calculer 10% et 90%).

En ce qui concerne la phase de montée (PM), dans certains modes de réalisation, l'étape de calcul (53) des paramètres pertinents dans l'évaluation comporte une étape (532) de calcul d'au moins un paramètre relatif à la vitesse (V) de montée des valeurs mesurées par les capteurs (10) de pression lors de la montée du sujet sur le dispositif (1). La figure 3B montre par une ellipse en pointillé la portion de la courbe sur laquelle pourra être calculée cette vitesse (V), à savoir toute la phase de montée (PM) dans cet exemple. Dans certains modes de réalisation, l'étape de calcul (53) des paramètres pertinents dans l'évaluation comporte une étape (531) de calcul d'au moins un paramètre relatif au délai (DAM) écoulé entre l'invitation à monter et la montée du sujet sur le dispositif (1).

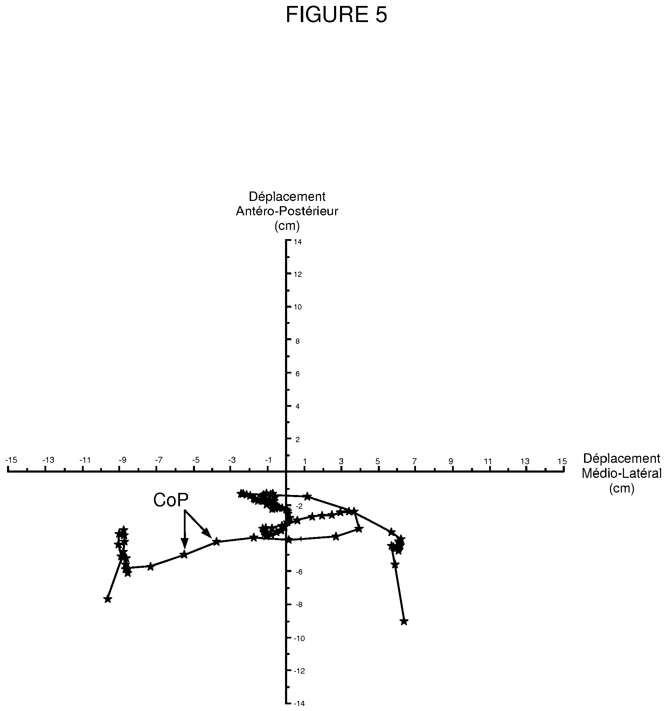

En ce qui concerne la phase de stabilité, l'étape de calcul (53) des paramètres pertinents dans l'évaluation, comme par exemple l'étape de calcul (533) des paramètres concernant la phase de stabilité (PS), comporte une étape de calcul (5300) du barycentre des forces verticales mesurées par chacun des capteurs (10) de pression pour déterminer la position du centre de pression (CoP, figure 5) correspondant à la projection du centre de gravité du sujet sur le plateau (13). Cette position du centre de pression (CoP) au cours du temps, pendant la phase de stabilité (PS), fournit un stabilogramme, dont un exemple est représenté sur la figure 5. Un tel stabilogramme permet la mise en oeuvre d'une étape de décomposition (5301) modale pour en extraire des fonctions modales intrinsèques. Les paramètres concernant cette phase de stabilité (PS) qui pourront être utilisés dans la présente invention pourront par exemple comporter au moins un paramètre déterminé sur ces fonctions modales intrinsèques. Par exemple, la représentation de ces fonctions modales intrinsèques dans un espace, dit des phases, peut prendre la forme d'ellipses dont on calcule la longueur (i.e., la plus grande dimension) et la largeur (la plus faible dimension). Cet exemple des dimensions d'ellipse ne doit pas être interprété de façon limitative bien que ce paramètre ait été déterminé comme étant particulièrement utile et l'invention couvre divers types de paramètres calculés sur une décomposition modale du signal. Ainsi, certains modes de réalisation pourront utiliser un ou plusieurs algorithmes de calcul pour la représentation dans l'espace des phases et le calcul de paramètres relatifs aux ellipses obtenues, notamment par exemple la longueur et/ou la largeur (qui sont pertinentes et dont le calcul requiert avantageusement peu de ressources).

Dans certains modes de réalisation, on pourra se focaliser uniquement sur la phase de montée PM (phase dynamique). On obtient ainsi un procédé (et un dispositif et un système) dans lequel on calcule par exemple au moins les paramètres relatifs à cette phase de montée (PM), comme par exemple les inflexions (pics et/ou sigmoïdes). On pourra également envisager un procédé dans lequel on calcule au moins les paramètres relatifs au délai avant montée (DAM) et/ou à la vitesse (V) de la montée décrits ci-avant. En effet, les inventeurs de la présente invention ont découvert que, même lors d'un protocole aussi simple qu'une montée du sujet sur un plateau de mesure, tel que celui d'un pèse-personne par exemple, le délai avant montée (DAM) ou la vitesse (V) de montée étaient des paramètres pertinents pour l'évaluation de l'équilibre du sujet. Ainsi, certains modes de réalisation de la présente invention sont des applications techniques de cette découverte par l'utilisation d'algorithmes relatifs à au moins un de ces paramètres.

Dans d'autres modes de réalisation, on pourra se focaliser uniquement sur la phase statique (PS). On obtient ainsi un procédé (et un dispositif et un système) dans lequel on calcule par exemple au moins les paramètres relatifs à cette phase statique (PS), comme par exemple les décompositions modales des stabilogrammes décrits ci-avant. On obtient ainsi par exemple un procédé d'évaluation de l'équilibre d'un sujet, caractérisé en ce qu'il est mis en œuvre à l'aide d'au moins un dispositif comportant un plateau (13) destiné à accueillir les pieds d'un sujet et monté sur une pluralité de capteurs (10) de pression mesurant des forces verticales appliquées sur le plateau (13). Le procédé peut alors comporter les étapes suivantes :

- Mesure (51), par les capteurs (10) de pression, des forces verticales appliquées sur le plateau (13), pendant la présence du sujet sur le dispositif (1),
- Calcul (53), à partir de ces valeurs mesurées, de paramètres pertinents dans l'évaluation, comprenant au moins une étape de calcul (52s) d'une phase, dite de stabilité (PS), des valeurs mesurées par les capteurs (10) de pression, lorsque le sujet est monté sur le dispositif (1), cette phase de stabilité (PS) étant déterminée comme la période démarrant après au moins un délai déterminé après l'invitation à montée sur le dispositif, puis au moins une étape de calcul (533) de paramètres concernant cette phase de stabilité (PS) incluant une étape de calcul (5300) du barycentre des forces verticales mesurées par chacun des capteurs (10) de pression pour déterminer la position du centre de pression (CoP) correspondant à la projection du centre de gravité du sujet sur le plateau (13) et fournissant un stabilogramme permettant la mise en oeuvre d'une étape de décomposition (5301) modale pour en extraire des fonctions modales intrinsèques, les paramètres concernant cette phase de stabilité (PS) comprenant au moins un paramètre déterminé sur ces fonctions modales intrinsèques.

Cependant, dans les modes de réalisation préférés, on combinera les calculs sur la phase statique (PS) et ceux sur la phase de montée (PM) car des études ont révélé que ces deux aspects sont à prendre en compte pour une évaluation de l'équilibre la plus pertinente possible. Ainsi, l'approche préférée fusionne des informations complémentaires liées à la qualité de l'équilibre statique et à la qualité de l'équilibre dynamique et les paramètres fournis ici sont particulièrement efficaces dans l'évaluation de l'équilibre.

On notera que l'on préfère ici que l'on réfère dans la présente demande à une invitation du sujet à monter sur le dispositif et que cette invitation permet de calculer le délai avant montée (DAM) du sujet, car elle fournit un temps de référence. Dans les modes de réalisation où ce calcul du délai (DAM) sera omis, cette invitation pourra l'être également. Ainsi, dans certains modes de réalisation, l'invention se dispense de cette caractéristique. D'une manière générale, les diverses étapes et/ou caractéristiques fournies dans la présente demande, notamment en référence à un mode de réalisation particulier, peuvent être isolées des autres étapes et/ou caractéristiques ou combinées avec d'autres étapes et/ou caractéristiques, à moins que l'inverse ne soit explicitement mentionné ou qu'il apparaisse que l'isolement ou la combinaison est impossible ou aboutit à une solution qui ne fonctionne pas.

D'autre part, on réfère ici principalement à cette invitation à monter et aux calculs réalisés à partir des mesures sur la phase de montée. Néanmoins, l'homme de métier comprendra à la lecture de la présente demande qu'il est possible également de réaliser des calculs sur la phase de descente (lorsque le sujet descend du dispositif). Par exemple, on pourra également déterminer des délais d'attente et des pourcentages de valeur de référence comme pour la phase de montée, pour déterminer une période définissant la phase de descente. Les paramètres calculés sur une telle phase de descente pourront par exemple être équivalents à ceux de la phase de montée, comme un délai avant descente (délai écoulé entre une invitation à descendre et la descente du sujet), une vitesse de descente (pente de décroissance des valeurs mesurées), la présence (et/ou les caractéristiques) d'inflexions (pics et/ou sigmoïdes) dans la descente, etc... D'autres paramètres pourront également être calculés sur cette phase de descente. En effet, les inventeurs ont observé de tels évènements lors des descentes et ont découvert qu'ils étaient pertinents, même dans le cas d'une descente d'un plateau tel que celui d'un pèse-personne, qui est généralement moins élevé que les obstacles utilisés dans les évaluations cliniques et qui est de ce fait adapté à une utilisation à domicile présentant moins de danger pour les sujets dont les risques de chute sont élevés.

La figure 4 représente schématiquement un exemple de procédé selon divers modes de réalisation de l'invention. Toutes les étapes y sont représentées, mais on comprendra de la présente description que certaines peuvent être omises. De même, on aura compris de ce qui précède que toutes ces étapes ne sont pas forcément mises en œuvre dans un seul et même dispositif (notamment en ce qui concerne les calculs qui peuvent être déportés comme détaillé ci-avant).

Ainsi, certains modes de réalisation du procédé comportent au moins une étape de transmission (54) des valeurs mesurées et/ou des paramètres calculés, par des moyens de communication (113) du dispositif (1), vers au moins un terminal communicant (2) et/ou au moins un serveur (3) de centralisation, via au moins un réseau de communication (RC). De même, certains modes de réalisation (non exclusifs des précédents) comportent au moins une étape de stockage (55) des valeurs mesurées et/ou des paramètres calculés dans des moyens de mémorisation (112, 212, 312).

De préférence, le procédé comporte au moins une étape de calcul (56) de comparaison des valeurs mesurées et/ou des paramètres calculés, stockés dans les moyens de mémorisation (112, 212, 312), pour au moins deux évaluations déterminées d'un même sujet, afin de permettre un suivi de l'évolution de la qualité de l'équilibre. Ainsi, que les données soient stockées dans des moyens de mémorisation (112) du dispositif (1), dans des moyens de mémorisation (212) d'un terminal (2) communicant ou dans des moyens de mémorisation (312) d'un serveur (3) de centralisation, le procédé pourra permettre une comparaison des performances d'un sujet par rapport à ses performances antérieures. On obtient ainsi un procédé permettant de détecter des dégradations de l'équilibre. L'invention prévoit d'ailleurs un traitement automatisé permettant des alertes automatiques (sur au moins un des dispositifs du système). Dans certains modes de réalisation, l'analyse des paramètres recueillis pourra nécessiter l'intervention d'un opérateur averti (par exemple un spécialiste de l'invention et/ou un médecin formé à son utilisation), par exemple sur un terminal (2). On notera que l'on n'a donné que les exemples d'un téléphone mobile et d'un ordinateur pour le terminal (2) mais que l'invention peut être implémentée dans d'autres dispositifs disposant de ressources électroniques suffisantes.

On notera que la présente invention effectue des calculs à partir de simples mesures de pression. Cela a été rendu possible grâce à la validation de la méthode, notamment en ce qui concerne le centre de pression (CoP). En effet, les centres de pression ne sont pas calculés dans la même façon en partant d'une plateforme de force et d'un pèse-personne. Les données d'une plateforme de force sont exprimées en moments et en forces pour les trois axes x, y, et z (Mx, My, Mz, Fx, Fy, Fz). Le centre de pression est calculé à partir des moments et des forces selon les formules ci-dessous :

$$AP = \frac{M_y}{F_z} \ , \ ML = \frac{-M_x}{F_z}$$

La force verticale de la plateforme de force est disponible directement, sans calculer (Fz).

Ne disposant pas de données sur les moments pour le pèse-personne, la méthode utilisée est fondamentalement différente. La trajectoire du centre de pression est déterminée en calculant le barycentre des forces verticales mesurées par les quatre capteurs du pèse-personne.

Il a fallu valider cette méthode en posant le dispositif (1) au centre d'une plateforme de force intégrée dans le sol, en compensant la force exercée par le dispositif (1) sur la plateforme grâce au pont de Wheatstone de cette plateforme et en comparant les mesures par les deux techniques et par diverses mesures expérimentales. Cette étude a permis de valider le procédé, le dispositif (1) et le système d'évaluation présentés dans la présente demande.

Des détails d'implémentation possibles sont fournis ci-après, de façon parfaitement illustrative et non limitative, en référence aux modes de réalisation où le dispositif (1) comporte des moyens de traitement de données pour la mise en œuvre de toutes les fonctionnalités décrites dans la présente demande (avec une carte pour chaque fonction). Cette liste détaille les différents moyens avec leur fonction :

Le capteur Infra-rouge réveille la carte de gestion/calcul dès qu'il détecte une présence, il utilise un fonctionnement par scrutation pour limiter la consommation.

La carte de traitement/mémorisation effectue les opérations :

- Mémorisation des données reçues, puis envoi des données mémorisées vers la carte Bluetooth via une liaison spécifique (par exemple RS232).
- Le début de la mémorisation commence avec la détection des données envoyées par la carte de gestion/calcul (présence de trame).
- La mémorisation s'arrête dès qu'il n'y a plus de trame ou bien lorsque la mémoire est pleine.
- Dès que la mémorisation s'arrête les données contenues en mémoire sont transmises sur la liaison spécifique pour être envoyée via Bluetooth.
- La taille maximum des données correspond à environ 15s de mesure, soit environ 4ko. Si le buffer est plein on passe à la transmission Bluetooth.

La carte de gestion/calcul effectue les opérations :

- contrôle des cartes de traitement/mémorisation, de mesure et d'affichage, gère le temps de mesure lorsque la personne est sur le pèse-personne, ainsi que le time out suivant :

    - Le capteur infra-rouge réveille la carte de gestion/calcul, il ne se passe plus rien après, il n'y a pas de détection de montée (la personne se présente sans monter, ou bien le capteur se déclenche sans la présence de la personne), on revient au standby.

    - S'il y a une erreur de Tare (entre les phases 1 et 2) alors on revient au standby.

- Gestion de l'alimentation des cartes de traitement/mémorisation, de mesure et d'affichage.
- Avec la carte de mesure, elle effectue la tare, envoie la trame d'initialisation à la carte de traitement/mémorisation.
- Elle affiche 0.0 et commande l'allumage de la LED verte pour indiquer que la personne peut monter.
- Elle récupère les mesures de la carte de mesure, calcule le temps et envoie ces données à la carte de traitement/mémorisation.
- Elle détecte le fait que la personne est montée, elle envoie l'affichage de "---" à la carte d'affichage, et commence le chronométrage du temps de mesure compris entre 5 et 10s, pendant ce temps elle envoie toujours les données des capteurs et le temps à la carte de traitement/mémorisation,
- A la fin du temps de mesure, elle calcule le poids, envoie le poids à la carte d'affichage et commande l'allumage de la LED rouge pour indiquer à la personne qu'elle peut descendre.
- Elle continue d'envoyer les valeurs des capteurs et le temps à la carte de traitement/mémorisation.
- Elle détecte le fait que 6a personne est descendue, arrête l'envoi des données capteurs, envoie le poids à la carte de traitement/mémorisation.
- Elle attend le signal de fin d'émission Bluetooth pour arrêter d'alimenter les cartes de mesure, d'affichage et de Traitement.
- Si la personne descend avant l'allumage de la LED rouge, le poids ne sera pas affiché et la carte de gestion/calcul enverra un code d'erreur dans la trame de poids,

La fréquence d'échantillonnage de la carte de mesure est de 16Hz.

La carte d'affichage intègre les LEDs rouge et verte.

Le pèse personne est alimenté par batterie. Lorsque la charge de la batterie devient trop faible, les LEDs rouge et verte clignotent au lieu de s'allumer en permanence lors de l'autorisation de montée et de descente, un message de batterie faible est ensuite envoyé via Bluetooth, lors d'un envoi de données.

Il existe 3 types de trames de données entre carte de gestion/calcul et carte de traitement/mémorisation :

- Trame d'initialisation (début de transmission) : données d'étalonnage des capteurs,
- Trame de données capteurs : valeurs des 4 capteurs, intervalle de temps entre deux mesures ;
- Trame de poids (fin de transmission) : octet, erreur, poids.

Des détails du protocole et des étapes d'affichage pour guider le sujet sont fournis ci-après, de façon parfaitement illustrative et non limitative :

Le cycle de la pesée débute lorsque la personne se positionne devant le pèse personne. Le capteur infrarouge détecte la posée du premier pied droit de la personne et débute la phase d'initialisation du pèse-personne. Des traits horizontaux sont affichés sur le cadran et indiquent à la personne qu'elle doit patienter.

Lorsque « 0.0 » s'affiche et la LED verte s'allume, l'acquisition des données des capteurs débute. La personne doit alors monter sur le pèse personne. Si le pèse-personne ne détecte aucun poids au bout de 15 secondes, le cycle est interrompu. Dès que le pèse-personne détecte une force minimale de 5 N, il affiche à nouveau des traits horizontaux sur le cadran et la LED verte s'éteint.

Après une attente de 10 secondes, le pèse-personne affiche le poids de la personne sur le cadran et la LED rouge s'allume. La personne peut alors descendre. L'acquisition des données des capteurs s'arrêtent lorsque la force exercée sur le pèse-personne descend en dessous de 5 N. La LED rouge s'éteint et le cadran affiche à nouveau des traits horizontaux.

Les données restent sauvegardées pendant dix minutes dans la mémoire de la deuxième carte. Si elles ne sont pas transférées dans le temps imparti, le pèse-personne s'éteint et les données sont alors perdues.

Des détails d'implémentation du transfert de données et de leur gestion ou utilisation ultérieure sont fournis ci-après, de façon parfaitement illustrative et non limitative :

Les données du pèse-personne sont transférées par l'intermédiaire d'une liaison Bluetooth. Selon le site de recueil des données, celles-ci sont enregistrées au niveau local, par exemple soit sur un PC (cas des centres pour lesquels plusieurs personnes seront suivies par le même système) soit dans un téléphone portable. Le logiciel de transfert est une application propriétaire. Les données sont réceptionnées puis transférées à un serveur grâce à un service Web.

Les données sont alors répertoriées dans une base de données suivant les identifiants du site d'évaluation et du sujet. Ces données capteur sont automatiquement utilisées afin de calculer la résultante verticale et les coordonnées de la trajectoire du centre de pression.

Ces trois types de données sont ensuite disponibles sur un site internet dont l'accès peut être protégé par login et mot de passe. Une fois connecté, l'utilisateur à accès à tous les fichiers enregistrés qu'il est habilité à consulter.

La sélection de fichiers peut être faite en utilisant un panneau de sélection qui permet de choisir des fichiers en fonction du site de mesure (domicile privée, EHPAD, etc.), du sujet, ou par le type de fichier (données du pèse-personne, données cliniques, etc.).

Une fois les données sélectionnées, il est possible de les télécharger.

En ce qui concerne le pèse-personne, il peut exister trois types de fichiers, par exemple au format CSV (Comma-separated values) pour une compatibilité facilitée:

- les fichiers BDPP qui contiennent les données brutes des quatre capteurs du pèse-personne
- les fichiers BVP qui contiennent les valeurs de la force résultante sur l'axe z
- les fichiers BCPMM qui contiennent les coordonnées de la trajectoire du centre de pression calculée par la méthode barycentrique

Les données sont alors transférées vers un PC ce qui permet d'afficher les résultats d'une pesée.

On comprend que la présente invention est particulièrement adaptée au suivi de l'évolution de l'équilibre d'un individu donné. Cependant, grâce à l'accumulation de données relatives à l'équilibre des individus, il est possible de constituer une base de données normative permettant l'utilisation de l'invention dans une évaluation ponctuelle de l'équilibre d'un individu.

L'invention peut être utilisée comme un outil non invasif scientifique pour caractériser quantitativement la performance du système de contrôle postural de l'homme. La technique utilisée ici a de nombreuses applications scientifiques, de recherche et pratiques. Par exemple, une base de données normative peut être établie, et à partir de cette base de données, des comparaisons peuvent être faites. Ces comparaisons pourraient être utilisées pour évaluer les changements de degré de stabilité posturale résultant de blessures, d'une maladie ou du processus de vieillissement.

D'autre part, la technique pourrait être utilisée dans les protocoles de rééducation. Par exemple, si le sujet a contracté une maladie ou a été blessé, le taux de récupération pourrait être suivi par la surveillance de l'équilibre grâce à l'invention.

De plus, la technique peut servir de base pour les techniques de biofeedback et de méthodologies visant à améliorer l'équilibre et la stabilité de l'individu.

Par ailleurs, l'invention pourrait être utilisé par les concepteurs de prothèses pour mesurer l'influence des prothèses sur la stabilité posturale.

De plus, il est envisagé que l'invention puisse être utilisée pour évaluer l'effet que divers médicaments ont sur l'équilibre d'un individu. Par exemple, un groupe de contrôle pourrait avoir reçu un placebo et un groupe d'étude pourrait recevoir un médicament particulier à l'étude. Une analyse de l'équilibre serait effectuée pour comparer les deux groupes et déterminer un effet éventuel du médicament sur le groupe d'étude.

Enfin, l'invention pourrait être employée par les services de neurologie des hôpitaux, des ministères et des programmes de gériatrie, maisons de retraite et les collectivités, les hôpitaux de soins préventifs et des centres médicaux, hôpitaux de réadaptation, les programmes spatiaux internationaux, les industries dont les travailleurs ont des risques de chute, les compagnies d'assurance, la recherche biomédicale, l'ingénierie et les programmes d'enseignement, la recherche en thérapie physique et les programmes d'enseignement, les écoles de médecine, de l'état et les services de police locaux (par exemple, des points de contrôle de sobriété), aux programmes sportifs, centres de conditionnement physique exercice et de sociétés de chaussures de sport.

L'annexe 1 montre un exemple illustratif et non limitatif des opérations réalisées au cours d'un protocole de mesures (dans l'exemple avec BlueTooth et avec les cartes décrites dans l'exemple fourni sur les détails d'implémentation ci-dessus). Sur cette annexe 1, la carte de mesure est désignée par la référence CE19, la carte d'affichage est désignée par la référence CE20 et la carte de gestion/calcul est désignée par la référence CUTT.

Il doit être évident pour les personnes versées dans l'art que la présente invention permet des modes de réalisation sous de nombreuses autres formes spécifiques sans l'éloigner du domaine d'application de l'invention comme revendiqué. Par conséquent, les présents modes de réalisation doivent être considérés à titre d'illustration, mais peuvent être modifiés dans le domaine défini par la portée des revendications jointes, et l'invention ne doit pas être limitée aux détails donnés ci-dessus.

## ANNEXE 1

## Revendications

1. Procédé de calcul de paramètres d'évaluation de l'équilibre d'un sujet, **caractérisé en ce qu'**il est mis en œuvre à l'aide d'au moins un dispositif comportant un plateau (13) destiné à accueillir les pieds d'un sujet et monté sur une pluralité de capteurs (10) de pression mesurant des forces verticales appliquées sur le plateau (13) lors de la montée du sujet sur le dispositif, et comporte les étapes suivantes :

- Mesure (51), par les capteurs (10) de pression, des forces verticales appliquées sur le plateau (13), au moins pendant la montée du sujet sur le dispositif (1),
- détermination (52d) d'une phase de montée (PM) des valeurs mesurées par les capteurs (10) de pression, lors de la montée du sujet sur le dispositif (1), cette phase de montée (PM) étant déterminée comme la période pendant laquelle lesdites valeurs se situent entre 10% et 90% d'une valeur de référence atteinte une fois le sujet monté sur le dispositif (1),
- Calcul (53), à partir de ces valeurs mesurées, d'au moins un paramètre relatif à au moins une sigmoïde (S) et/ou au moins un pic (P) dans ladite phase de montée (PM).

2. Procédé selon la revendication 1, **caractérisé en ce que** l'étape de calcul (53) comporte une étape (530) de calcul de la somme des valeurs mesurées par chacun des capteurs (10) de pression.

3. Procédé selon une des revendications 1 et 2, **caractérisé en ce qu'**il comporte une étape de diffusion (50), par des moyens de diffusion (12), d'informations relatives au moins à une invitation du sujet à monter sur le dispositif (1), l'étape de calcul (53) comportant une étape (531) de calcul d'au moins un paramètre relatif au délai (DAM) écoulé entre l'invitation à monter et la montée du sujet sur le dispositif (1).

4. Procédé selon une des revendications 1 à 3, **caractérisé en ce que** l'étape de calcul (53) comporte une étape (532) de calcul d'au moins un paramètre relatif à la vitesse (V) de montée des valeurs mesurées par les capteurs (10) de pression lors de la montée du sujet sur le dispositif (1).

5. Procédé selon une des revendications 1 à 4, **caractérisé en ce que** l'étape de calcul (53) est précédée d'une étape de calcul (52s) d'une phase, dite de stabilité (PS), des valeurs mesurées par les capteurs (10) de pression, lorsque le sujet est monté sur le dispositif (1), cette phase de stabilité (PS) étant déterminée comme la période démarrant à un délai déterminé (DO), dit d'attente, après que lesdites valeurs mesurées aient atteint 90% de la valeur de référence et se terminant à un délai (DS) déterminé, dit de stabilité, après ledit délai d'attente (DO), l'étape de calcul (53) comprenant au moins une étape de calcul (533) de paramètres concernant cette phase de stabilité (PS).

6. Procédé selon la revendication 5, **caractérisé en ce que** l'étape de calcul (533) des paramètres concernant la phase de stabilité (PS) comporte une étape de calcul (5300) du barycentre des forces verticales mesurées par chacun des capteurs (10) de pression pour déterminer la position du centre de pression (CoP) correspondant à la projection du centre de gravité du sujet sur le plateau (13).

7. Procédé selon la revendication 6, **caractérisé en ce que** l'étape de calcul (5300) de la position du centre de pression (CoP) au cours du temps, pendant la phase de stabilité (PS), fournit un stabilogramme permettant la mise en œuvre d'une étape de décomposition (5301) modale pour en extraire des fonctions modales intrinsèques, les paramètres concernant cette phase de stabilité (PS) comprenant au moins un paramètre déterminé sur ces fonctions modales intrinsèques.

8. Procédé selon une des revendications 1 à 7, **caractérisé en ce qu'**il comporte au moins une étape de transmission (54) des valeurs mesurées et/ou des paramètres calculés, par des moyens de communication (113) du dispositif (1), vers au moins un terminal communicant (2) et/ou au moins un serveur (3) de centralisation, via au moins un réseau de communication (RC).

9. Dispositif (1) d'évaluation de l'équilibre d'un sujet, **caractérisé en ce qu'**il comporte un plateau (13) destiné à accueillir les pieds d'un sujet et monté sur une pluralité de capteurs (10) de pression permettant de mesurer des forces verticales appliquées sur le plateau (13), et des moyens de traitement de données (11) agencés pour la mise en œuvre du procédé selon l'une des revendications 1 à 8.

10. Dispositif (1) selon la revendication 9, **caractérisé en ce que** le plateau (13) possède des dimensions adaptées à la taille moyenne des pieds des sujets, de façon à s'affranchir de problèmes liés à la position des pieds des sujets sur le plateau lors des mesures.

11. Dispositif (1) selon une des revendications 9 et 10, **caractérisé en ce qu'**il comporte des moyens d'affichage (12) positionnés sur le dispositif (1) de sorte qu'un sujet debout sur le plateau (13) puisse voir l'affichage, de façon à ce que la posture des sujets utilisant le dispositif (1) soit standardisée.

**12.** Système d'évaluation de l'équilibre d'un sujet, **caractérisé en ce qu'**il comporte un dispositif (1) comprenant un plateau (13) destiné à accueillir les pieds d'un sujet et monté sur une pluralité de capteurs (10) de pression permettant de mesurer des forces verticales appliquées sur le plateau (13), et des moyens de traitement de données (11) agencés pour recueillir dynamiquement les valeurs mesurées par les capteurs (10) de pression au moins avant et pendant la montée du sujet sur le dispositif (1) et comprenant des moyens de communication de données (113) agencés pour transmettre les valeurs mesurées vers au moins un terminal communicant (2) comprenant des moyens de communication (213) agencés pour recevoir les valeurs mesurées par le dispositif (1) et les transmettre à au moins un serveur (3) de centralisation dont des moyens de traitement de données (31) sont agencés pour la mise en œuvre d'au moins une des étapes de calcul (52d, 52s, 53, 56) du procédé selon l'une des revendications 1 à 8.

**13.** Système d'évaluation de l'équilibre d'un sujet, **caractérisé en ce qu'**il comprend un dispositif (1) d'évaluation de l'équilibre d'un sujet, comportant un plateau (13) destiné à accueillir les pieds d'un sujet et monté sur une pluralité de capteurs (10) de pression permettant de mesurer des forces verticales appliquées sur le plateau (13), et des moyens de traitement de données (11) agencés pour recueillir dynamiquement les valeurs mesurées par les capteurs (10) de pression au moins avant et pendant la montée du sujet sur le dispositif (1) et comprenant des moyens de communication de données (113) agencés pour transmettre les valeurs mesurées vers au moins un terminal communicant (2) comprenant des moyens de communication (213) agencés pour recevoir les valeurs mesurées par le dispositif (1) et des moyens de traitement de données (21) sont agencés pour la mise en œuvre d'au moins une des étapes de calcul (52d, 52s, 53, 56) du procédé selon l'une des revendications 1 à 8, les moyens de communication (213) de ce terminal communicant (2) étant également agencés pour transmettre les valeurs mesurées et/ou les paramètres calculés vers au moins un serveur (3) de centralisation.

**Patentansprüche**

**1.** Verfahren zur Berechnung von Parametern zur Bewertung des Gleichgewichts eines Subjekts, **dadurch gekennzeichnet, dass** es mittels mindestens einer Vorrichtung durchgeführt wird, die eine Platte (13) umfasst, die dazu vorgesehen ist, die Füße eines Subjekts aufzunehmen, und auf mehreren Drucksensoren (10) montiert ist, die während des Betretens der Vorrichtung durch das Subjekt auf die Platte (13) ausgeübte vertikale Kräfte messen, und umfassend die folgenden Schritte:

- Messen (51) von auf die Platte (13) ausgeübten vertikalen Kräften mindestens während des Betretens der Vorrichtung (1) durch das Subjekt durch die Drucksensoren (10),
- Bestimmen (52d) einer Betretphase (PM) aus durch die Drucksensoren (10) während des Betretens der Vorrichtung (1) durch das Subjekt gemessenen Werten, wobei diese Betretphase (PM) als der Zeitraum bestimmt wird, während dem die Werte sich zwischen 10 % und 90 % eines Referenzwerts befinden, der einmal erreicht wurde, als das Subjekt die Vorrichtung (1) betrat,
- Berechnen (53) mindestens eines Parameters in Bezug auf mindestens ein Sigmoid (S) und/oder mindestens einer Spitze (P) in der Betretphase (PM) aus diesen gemessenen Werten.

**2.** Verfahren nach Anspruch 1, **dadurch gekennzeichnet, dass** der Schritt des Berechnens (53) einen Schritt (530) eines Berechnens der Summe von durch jeden der Drucksensoren (10) gemessenen Werten umfasst.

**3.** Verfahren nach einem der Ansprüche 1 und 2, **dadurch gekennzeichnet, dass** es einen Schritt eines Verbreitens (50) von Informationen in Bezug auf mindestens eine Einladung des Subjekts, die Vorrichtung (1) zu betreten, durch Verbreitungsmittel (12) umfasst, wobei der Schritt des Berechnens (53) einen Schritt (531) eines Berechnens von mindestens einem Parameter in Bezug auf eine Zeitspanne (DAM), die zwischen der Einladung zum Betreten und dem Betreten der Vorrichtung (1) durch das Subjekt verstrichen ist, umfasst.

**4.** Verfahren nach einem der Ansprüche 1 bis 3, **dadurch gekennzeichnet, dass** der Schritt des Berechnens (53) einen Schritt (532) eines Berechnens von mindestens einem Parameter in Bezug auf die Geschwindigkeit (V) des Betretens aus durch die Drucksensoren (10) während des Betretens der Vorrichtung (1) durch das Subjekt gemessenen Werten umfasst.

**5.** Verfahren nach einem der Ansprüche 1 bis 4, **dadurch gekennzeichnet, dass** dem Schritt des Berechnens (53) einen Schritt eines Berechnens (52s) einer Phase, einer so genannten Stabilitätsphase (PS), aus durch die Drucksensoren (10) gemessenen Werten, wenn das Subjekt die Vorrichtung (1) betritt, umfasst, wobei diese Stabilitätsphase (PS) als der Zeitraum bestimmt wird, der mit einer bestimmten Zeitspanne (DO), einer so genannten Wartezeitspanne, beginnt, nach der die gemessenen Werte 90 % des Referenzwerts erreichten, und der mit einer bestimmten Zeitspanne (DS), einer so genannten Stabilitätszeitspanne, nach der Wartezeitspanne (DO) endet, wobei der Schritt des Berechnens (53) mindestens einen Schritt eines Berechnens (533) von Parametern, die diese Stabilitätsphase (PS) betreffen, umfasst.

6. Verfahren nach Anspruch 5, **dadurch gekennzeichnet, dass** der Schritt des Berechnens (533) von Parametern, die die Stabilitätsphase (PS) betreffen, einen Schritt eines Berechnens (5300) des Baryzentrums von durch jeden der Drucksensoren (10) gemessenen vertikalen Kräften umfasst, um die Position des Druckzentrums (CoP), das der Projektion des Schwerpunkts des Subjekts auf der Platte (13) zu bestimmen.

7. Verfahren nach Anspruch 6, **dadurch gekennzeichnet, dass** der Schritt des Berechnens (5300) der Position des Druckzentrums (CoP) im Laufe der Zeit während der Stabilitätsphase (PS) ein Stabilogramm bereitstellt, das das Durchführen eines modalen Zerlegungsschritts (5301) ermöglicht, um daraus intrinsische modale Funktionen zu extrahieren, wobei die Parameter diese Stabilitätsphase (PS) betreffen, umfassend mindestens einen an diesen intrinsischen modalen Funktionen bestimmten Parameter.

8. Verfahren nach einem der Ansprüche 1 bis 7, **dadurch gekennzeichnet, dass** es mindestens einen Schritt eines Übertragens (54) von gemessenen Werten und/oder berechneten Parametern durch Kommunikationsmittel (113) der Vorrichtung (1) an mindestens ein Kommunikationsendgerät (2) und/oder mindestens einen Zentralisierungsserver (3) mittels mindestens einem Kommunikationsnetz (RC) umfasst.

9. Vorrichtung (1) zur Bewertung des Gleichgewichts eines Subjekts, **dadurch gekennzeichnet, dass** sie eine Platte (13), die dazu vorgesehen ist, die Füße eines Subjekts aufzunehmen, und auf mehreren Drucksensoren (10) montiert ist, die ermöglichen, auf die Platte (13) ausgeübte vertikale Kräfte zu messen, und Datenverarbeitungsmittel (11), die dazu eingerichtet sind, das Verfahren nach einem der Ansprüche 1 bis 8 durchzuführen, umfasst.

10. Vorrichtung (1) nach Anspruch 9, **dadurch gekennzeichnet, dass** die Platte (13) über Abmessungen verfügt, die auf die durchschnittliche Größe von Füßen von Subjekten angepasst sind, um Probleme zu beheben, die mit der Position von Füßen von Subjekten auf der Platte während Messungen verbunden sind.

11. Vorrichtung (1) nach einem der Ansprüche 9 und 10, **dadurch gekennzeichnet, dass** sie Anzeigemittel (12) umfasst, die auf der Vorrichtung (1) positioniert sind, so dass ein Subjekt, das auf der Platte (13) steht, die Anzeige sehen kann, so dass die Haltung von Subjekten, die die Vorrichtung (1) verwenden, standardisiert wird.

12. System zur Bewertung des Gleichgewichts eines Subjekts, **dadurch gekennzeichnet, dass** sie eine Vorrichtung (1) umfasst, die eine Platte (13), die dazu vorgesehen ist, die Füße eines Subjekts aufzunehmen, und auf mehreren Drucksensoren (10) montiert ist, die ermöglichen, auf die Platte (13) ausgeübte vertikale Kräfte zu messen, und Datenverarbeitungsmittel (11) umfasst, die dazu eingerichtet sind, die von den Drucksensoren (10) mindestens vor und während des Betretens der Vorrichtung (1) durch das Subjekt gemessenen Werte dynamisch zu erfassen, und Datenkommunikationsmittel (113) umfassen, die dazu eingerichtet sind, die gemessenen Werte an mindestens ein Kommunikationsendgerät (2) zu übertragen, das Kommunikationsmittel (213) umfasst, die dazu eingerichtet sind, die von der Vorrichtung (1) gemessenen Werte zu empfangen und sie an mindestens einen Zentralisierungsserver (3) zu übertragen, dessen Datenverarbeitungsmittel (31) dazu eingerichtet sind, mindestens einen der Schritte des Berechnens (52d, 52s, 53, 56) des Verfahrens nach einem der Ansprüche 1 bis 8 durchzuführen.

13. System zur Bewertung des Gleichgewichts eines Subjekts, **dadurch gekennzeichnet, dass** sie eine Vorrichtung (1) zur Bewertung des Gleichgewichts eines Subjekts umfasst, die eine Platte (13), die dazu vorgesehen ist, die Füße eines Subjekts aufzunehmen, und auf mehreren Drucksensoren (10) montiert ist, die ermöglichen, auf die Platte (13) ausgeübte vertikale Kräfte zu messen, und Datenverarbeitungsmittel (11) umfasst, die dazu eingerichtet sind, die von den Drucksensoren (10) mindestens vor und während des Betretens der Vorrichtung (1) durch das Subjekt gemessenen Werte dynamisch zu erfassen, und Datenkommunikationsmittel (113) umfassen, die dazu eingerichtet sind, die gemessenen Werte an mindestens ein Kommunikationsendgerät (2) zu übertragen, das Kommunikationsmittel (213) umfasst, die dazu eingerichtet sind, die von der Vorrichtung (1) gemessenen Werte zu empfangen, und wobei Datenverarbeitungsmittel (21) dazu eingerichtet sind, mindestens einen der Schritte des Berechnens (52d, 52s, 53, 56) des Verfahrens nach einem der Ansprüche 1 bis 8 durchzuführen, wobei die Kommunikationsmittel (213) dieses Kommunikationsendgeräts (2) außerdem dazu eingerichtet sind, die gemessenen Werte und/oder die berechneten Parameter an mindestens einen Zentralisierungsserver (3) zu übertragen.

**Claims**

1. Method for calculating parameters for evaluating the equilibrium of a subject, **characterised in that** it is carried out with the aid of at least one device comprising a platform (13) designed to accommodate the feet of a subject and mounted on a plurality of pressure sensors (10) that measure vertical forces applied to the platform (13) when the subject steps on the device, and comprises the following steps:

   - measurement (51), by the pressure sensors (10), of the vertical forces applied to the platform (13), at least while the subject steps on the device (1),
   - determination (52d) of a phase of increase (PM) in the values measured by the pressure sensors (10), when the subject steps on the device (1), this phase of increase (PM) being determined as the period during which said values are between 10% and 90% of a reference value attained once the subject has stepped on the device (1),
   - calculation (53), on the basis of these measured values, of at least one parameter relating to at least one sigmoid (S) and/or at least one peak (P) in said phase of increase (PM).

2. Method according to claim 1, characterised that the calculation step (53) comprises a step (530) of calculation of the sum of values measured by each of the pressure sensors (10).

3. Method according to one of claims 1 and 2, **characterised in that** it comprises a step of dissemination (50), by dissemination means (12), of information relating to at least one invitation to the subject to step on the device (1), the calculation step (53) comprising a step (531) of calculating at least one parameter relating to the time (DAM) that has passed between the invitation to step on and the subject stepping on the device (1).

4. Method according to one of claims 1 to 3, **characterised in that** the calculation step (53) comprises a step (532) of calculating at least one parameter relating to the speed (V) of increase in the values measured by the pressure sensors (10) when the subject steps on the device (1).

5. Method according to one of claims 1 to 4, **characterised in that** the calculation step (53) is preceded by a step of calculation (52s) of a phase, called the stability phase (PS), of the values measured by the pressure sensors (10), when the subject has stepped on the device (1), this stability phase (PS) being determined as the period beginning at a given time (DO), called the waiting time, after said measured values have reached 90% of the reference value and ending at a given time (DS), called the stability time, after said waiting time (DO), the calculation step (53) comprising at least one step of calculation (533) of parameters concerning this stability phase (PS).

6. Method according to claim 5, **characterised in that** the step of calculation (533) of the parameters concerning the stability phase (PS) comprises a step of calculation (5300) of the barycentre of the vertical forces measured by each of the pressure sensors (10) in order to determine the position of the centre of pressure (CoP) corresponding to the projection of the centre of gravity of the subject on the platform (13).

7. Method according to claim 6, **characterised in that** the step of calculation (5300) of the position of the centre of pressure (CoP) over time, during the stability phase (PS), provides a stabilogram making it possible to carry out a step of modal decomposition (5301) in order to extract intrinsic modal functions therefrom, the parameters concerning this stability phase (PS) comprising at least one parameter determined from these intrinsic modal functions.

8. Method according to one of claims 1 to 7, **characterised in that** it comprises at least one step of transmission (54) of the measured values and/or calculated parameters, by communication means (113) of the device (1), to at least one communicating terminal (2) and/or at least one centralisation server (3), via at least one communication network (RC).

9. Device (1) for evaluating the equilibrium of a subject, **characterised in that** it comprises a platform (13) designed to accommodate the feet of a subject and mounted on a plurality of pressure sensors (10) that make it possible to measure vertical forces applied to the platform (13), and data processing means (11) arranged to carry out the method according to one of claims 1 to 8.

10. Device (1) according to claim 9, **characterised in that** the platform (13) has dimensions adapted to the average size of the feet of subjects, so as to deal with problems connected with the position of the feet of the subjects on the platform during measurements.

**11.** Device (1) according to one of claims 9 and 10, characterised that it comprises display means (12) positioned on the device (1) so that a subject standing on the platform (13) can see the display, in such a way that the posture of subjects using the device (1) is standardised.

**12.** System for evaluating the equilibrium of a subject, **characterised in that** it comprises a device (1) including a platform (13) designed to accommodate the feet of a subject and mounted on a plurality of pressure sensors (10) that make it possible to measure vertical forces applied to the platform (13), and data processing means (11) arranged to obtain dynamically the values measured by the pressure sensors (10) at least before and while the subject steps on the device (1) and including data communication means (113) arranged to transmit the measured values to at least one communicating terminal (2) including communication means (213) arranged to receive the values measured by the device (1) and transmit them to at least one centralisation server (3) of which the data processing means (31) are arranged to carry out at least one of the calculation steps (52d, 52s, 53, 56) of the method according to one of claims 1 to 8.

**13.** System for evaluating the equilibrium of a subject, **characterised in that** it includes a device (1) for evaluating the equilibrium of a subject, comprising a platform (13) designed to accommodate the feet of a subject and mounted on a plurality of pressure sensors (10) that make it possible to measure vertical forces applied to the platform (13), and data processing means (11) arranged to obtain dynamically the values measured by the pressure sensors (10) at least before and while the subject steps on the device (1) and including data communication means (113) arranged to transmit the measured values to at least one communicating terminal (2) including communication means (213) arranged to receive the values measured by the device (1) and data processing means (21) are arranged to carry out at least one of the calculation steps (52d, 52s, 53, 56) of the method according to one of claims 1 to 8, the communication means (213) of this communicating terminal (2) also being arranged to transmit the measured values and/or the calculated parameters to at least one centralisation server (3).

# FIGURE 1

# FIGURE 2

Communication

Traitement/
Mémorisation

Capteur IR

Mesure

Gestion/
Calcul

Affichage

## FIGURE 3A

## FIGURE 3B

50 — Affichage ⟷ Mesure — 51

52d — Calcul PM          Calcul PS — 52s

53

530 — Calcul Somme

531 — Calcul DAM

532 — Calcul V

533 — Calcul paramètres PS

5300 — Calcul Barycentre

5301 — Décomposition Modale

54 — Transmission ⟷ Stockage — 55

56 — Comparaison

# FIGURE 5

Déplacement
Antéro-Postérieur
(cm)

Déplacement
Médio-Latéral
(cm)

CoP

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- JP H9257556 B

- FR 0205382

**Littérature non-brevet citée dans la description**

- **DE MICHEL-PELLEGRINO et al.** Effect of aging on the weight transfer phase when stepping-down backwards. *Gait & Posture,* 2006, vol. 24S, S281-S282
- **DE MICHEL-PELLEGRINO et al.** Evaluation of the risk of falling in institution-dwelling elderly: clinical tests versus biomechanical analysis of stepping-up. *Proc. 2007 annual international conférence of the IEEE Engineering in Medicine and Biology Society,* 6121-6124

- **DE JONSSON et al.** Age-related différences in postural adjustments in connection with différent tasks involving weight transfer while standing. *Gait & Posture,* 2007, vol. 26, 508-515
- **DE SNOUSSI et al.** Reconstructed Phase Spaces of Intrinsic Mode Functions. Application to Postural Stability Analysis. *Proc. 28th Annual International Conference of the IEEE Engineering in Medicine and Biology Society,* 2006, 4584-4589
- **DE MICHEL et al.** The effect of age on dynamic postural equilibrium when stepping up. *Gait & Posture,* 2005, vol. 21, S9-S10